# EUROPEAN PATENT APPLICATION

(11) **EP 4 238 497 A1**
(43) Date of publication of application: **06.09.2023**
(21) Application number: 21884537.8
(22) Date of filing: 29.07.2021
(51) Int. Cl.: A61B 5/287, A61B 5/318, A61B 5/00

(54) **ELECTROPHYSIOLOGICAL MAPPING CATHETER APPARATUS**

(30) Priority: 29.10.2020 CN 202022458290 U
(71) Applicant: SUZHOU SINUS MEDICAL TECHNOLOGIES CO., LTD, Suzhou , Jiangsu 215000 (CN)
(72) Inventor: LIU, Xingpeng, Shenzhen, Guangdong 518122 (CN); SONG, Jingzhong, Shenzhen, Guangdong 518122 (CN); ZHANG, Mingfang, Shenzhen, Guangdong 518122 (CN); LUO, Xiaoping, Shenzhen, Guangdong 518122 (CN); MAO, Jun, Shenzhen, Guangdong 518122 (CN)
(74) Representative: Sackin, Robert
(86) International application number: PCT/CN2021/109289
(87) International publication number: WO 2022/088802

(57) **Abstract**

An electrophysiological mapping catheter device (10) includes a catheter, an adjusting handle device (300), electrodes (400), and a connector (500). The catheter includes an adjusting tube (200) and a plurality of branch tubes (100). Two ends of each branch tube (100) are respectively a connection end (120) and a detection end (110). The detection ends (110) are arranged in a staggered manner. The electrodes (400) are respectively disposed on the detection ends (110), and the connection ends (120) are connected to the adjusting tube (200). The adjusting tube (200) is connected to the adjusting handle device (300). The adjusting handle device (300) is capable of adjusting a bending angle of the adjusting tube (200). The connector (500) is disposed on the adjusting handle device (300) and is electrically connected to the electrodes (400). The connector (500) is further configured to be connected with an electrophysiological system for transmitting electrical signals detected by the electrodes (400). The electrophysiological mapping catheter device (10) can adapt to an irregular heart chamber space and record electrophysiological signals from numerous folds or loose pits on the endocardial surface. Moreover, a more accurate three-dimensional mapping image can be formed in a relatively short time, reducing the difficulty of identification by surgeons and facilitating the operation.

## Description

### TECHNICAL FIELD

The present utility model relates to medical devices, and particularly to an electrophysiological mapping catheter device.

### BACKGROUND

Electrophysiological mapping catheters are very important in the field of cardiac electrophysiology and can be used for recording the electrophysiological signals in the body. Thereby, an abnormal lesion can be found out, and an ablation can be guided. After the ablation, the therapeutic effect can be verified by using the electrophysiological mapping catheter. The application of the electrophysiological mapping catheters is more and more prevalent with the development of intracardiac electrophysiology and corresponding medical devices. Intracardiac synchronous multipoint mapping is a new emerging technique used for mapping of various complex arrhythmias and radiofrequency catheter ablation.

A three-dimensional mapping system can display three-dimensional anatomical images, electrocardiosignals, etc., within the heart chambers, and has been widely used in the field of clinical cardiac electrophysiology due to its unique safety, accuracy and high efficiency. However, a current three-dimensional mapping system has certain limitations in practical applications, due to the following reasons. The spatial structure of the heart chambers does not have a regular shape, the sizes of the human hearts are varied, and individual differences do exist. Moreover, the intracardiac physiological structure is complex, the endocardial surface is not smooth, and there are some uneven striped sulci or loose pits. As for the sulci formed by the pectinate muscles in the atria and loose meshwork formed by the trabeculae muscles in the ventricles, it is difficult for the tip end portion of a current electrophysiological mapping catheter to sufficiently attach them by electrode. Therefore, the electrophysiological signals at these complex anatomical structures cannot be obtained, and thus the intracardiac electrophysiological diagnosis and treatment cannot be completely satisfied. It can be seen that as for an irregular heart chamber structure, a non-smooth inner wall, as well as a narrow space at a proximal or distal end, etc., a current three-dimensional mapping system cannot make contact with them sufficiently, so that it is difficult to accurately record the electrical signals thereof.

### SUMMARY

In view of the above, there is a need to provide an electrophysiological mapping catheter device, which can adapt to the heart chambers with an irregular space and record electrophysiological signals from numerous folds or loose sulci on the endocardial surface. Moreover, a more accurate three-dimensional mapping image can be formed in a relatively short time, reducing the difficulty of identification by surgeons and facilitating the operation.

An electrophysiological mapping catheter device includes a catheter, an adjusting handle device, electrodes, and a connector. The catheter includes an adjusting tube and a plurality of branch tubes. The two ends of the branch tube are respectively a connection end and a detection end. The plurality of detection ends are arranged in a staggered manner. The electrodes are respectively disposed on the detection ends. The connection ends are connected to the adjusting tube. The adjusting tube is connected to the adjusting handle device. The adjusting handle device is capable of adjusting a bending angle of the adjusting tube. The connector is disposed on the adjusting handle device and is electrically connected to the electrodes. The connector is further configured to be connected with an electrophysiological system for transmitting electrical signals detected by the electrodes.

In an embodiment, the end surfaces of the detection ends of the plurality of branch tubes are disposed in a same curved surface.

In an embodiment, the curved surface is a spherical surface, a partial spherical surface, an elliptical surface, or a partial elliptical surface.

In an embodiment, an axial angle between the branch tubes and the adjusting tube is 10° to 180°.

In an embodiment, the branch tubes are flexible and have shape memory capability.

In an embodiment, the plurality of branch tubes are divided into at least two groups, and the branch tubes in each group are symmetrically distributed with respect to an axial direction of the adjusting tube.

In an embodiment, the adjusting tube includes a distal end portion connected to the branch tubes and a proximal end portion connected to the adjusting handle device. The electrophysiological mapping catheter device further includes an adjusting pulling wire. One end of the adjusting pulling wire is connected to the distal end portion, and the other end of the adjusting pulling wire is connected to the adjusting handle device.

In an embodiment, the distal end portion is flexible.

In an embodiment, the distal end portion includes a first tube chamber. One opening end of the first tube chamber is disposed at an end surface of the distal end portion, and the other end of the first tube chamber is in communication with a perfusion tube disposed in the proximal end portion. The perfusion tube is extended to the adjusting handle device and is in communication with an extension tube. The extension tube is connected to the adjusting handle device and is configured for connecting with an external perfusion pump.

In an embodiment, the distal end portion includes a second tube chamber. One end of the second tube chamber is in communication with the distal end portion, and the other end of the second tube chamber is in communication with the proximal end portion. One end of the adjusting pulling wire is connected to the distal end portion, and the other end of the adjusting pulling wire is passed through the second tube chamber and connected to the adjusting handle device.

In an embodiment, the distal end portion further includes a third tube chamber. One end of the third tube chamber is in communication with the branch tubes, and the other end of the third tube chamber is in communication with the proximal end portion. Conducting wires of the electrodes are sequentially passed through the third tube chamber, the proximal end portion, and the adjusting handle device, and connected to the connector.

In an embodiment, the electrodes are ring-shaped electrodes. The width of each ring electrode is 0.2 to 2 mm. The outer diameter of each ring electrode is 0.3 to 1 mm. A distance between adjacent ring electrodes is 1 to 10 mm. Inner surfaces of the ring-shaped electrodes are respectively connected to conducting wires. The conducting wires are insulated from each other and connected to the connector.

The electrophysiological mapping catheter device of the present utility model can adapt to the irregular heart chamber space and record electrophysiological signals from numerous folds or loose sulci on the endocardial surface. Moreover, a more accurate three-dimensional mapping image can be formed in a relatively short time, reducing the difficulty of identification by surgeons and facilitating the operation. The electrophysiological mapping catheter device of the present utility model can make sufficient contact with an irregular heart chamber structure, a non-smooth inner wall, as well as a narrow space at a proximal or distal end etc., and thus can accurately record the electrical signals therefrom. The plurality of branch tubes of the electrophysiological mapping catheter device of the present utility model form a divergent spatial structure with the detection ends arranged a staggered manner. The staggered detection ends can adapt to various irregular heart chamber structures, and the electrodes of the detection ends can readily access to various narrow spaces and microstructures in the heart chamber.

Compared with the prior art, the plurality of branch tubes of the electrophysiological mapping catheter device of the present utility model form a divergent structure with adjustable angles. The numbers of the branch tubes and the electrodes are relatively large. Therefore, the reliability of the contact between the electrodes and the irregular heart chamber, or between the electrodes and folds or sulci on the inner wall of the heart chamber during operation is improved, and thus the precision and accuracy of electrophysiological signal acquisition is improved, thereby reducing the operation time, making the operation safer and more efficient, and providing great convenience for the surgeons.

### BRIEF DESCRIPTION OF THE DRAWINGS

FIG. 1 is an overall schematic structural view of an electrophysiological mapping catheter device according to an embodiment of the present utility model.
FIG. 2 is a schematic view of branch tubes arranged in a spherical configuration of the electrophysiological mapping catheter device shown in FIG. 1.
FIG. 3 is a schematic projection view of the electrophysiological mapping catheter device along direction A of FIG. 1.
FIG. 4 is a schematic view of the branch tubes arranged in a hemispherical configuration of the electrophysiological mapping catheter device shown in FIG. 1.
FIG. 5 is a schematic projection view of the electrophysiological mapping catheter device along direction A of FIG. 4.
FIG. 6 is a schematic view of electrodes in the branch tubes of the electrophysiological mapping catheter device shown in FIG. 1.
FIG. 7 is a schematic cross-sectional view of an adjusting tube of the electrophysiological mapping catheter device shown in FIG. 1.

### Reference signs:

10, electrophysiological mapping catheter device; 100, branch tube; 110, 110a, 110b, 110c, 110d, 110e, 110f, 110g, 110h, 110i, 110A, 110B, 110C, 110D, 110E, 110F, 110G, 110H, 110I, detection end; 120, connection end; 200, adjusting tube; 210, distal end portion; 211, first tube chamber; 212, second tube chamber; 213, third tube chamber; 214, outer tube; 215, intermediate reinforcement metal layer; 216, inner tube; 220, proximal end portion; 300, adjusting handle device; 400, electrode; 500, connector; 600, conducting wire; 700, extension tube; and 800, Luer taper.

### DETAILED DESCRIPTION

The present utility model will now be described in detail with reference to the accompanying drawings for convenience of understanding the present utility model. The preferred embodiments of the present utility model are shown in the accompanying drawings. However, the present utility model can be implemented in many different forms and is not limited to the embodiments described herein. In contrast, these embodiments are provided to facilitate a more thorough understanding of the disclosure of the utility model.

In the description of the utility model, it should be understood that an orientation or positional relationship indicated by the terms "center", "up", "down", "bottom ", "inside", "outside", etc., are based on the orientation or positional relationship shown in the accompanying drawings, for ease and simplification of description of the present utility model, rather than indicating or implying the device or member referred to must have a particular orientation, or be configured and operated in a particular orientation, and therefore should not be construed as a limitation to the present utility model.

It should be understood that the elements described by the terms "the first", "the second", etc., should not be limited to these terms, which are used only to distinguish the same type of elements from one another. For example, without departing from the scope of the present utility model, "the first" element may also be named "the second" element. Similarly, "the second" element may also be named "the first" element.

In the description of the present utility model, it should be noted that unless otherwise specified and defined, the terms "connect", "communicate", etc., shall be understood in a broad sense. For example, it can be a fixed connection, a removable connection, or an integrally connection; it can be a mechanical connection, or an electrical connection; and it can be a direct connection, or an indirect connection with an intermediate medium, or an internal communication of the two elements. That is, when one element is referred to be "fixed" to the other element, it may be directly fixed to the other element, or there is an intermediate element. When one element is considered to be "connected" with the other element, it may be directly "connected" with the other element or there may be an intermediate element. For those of ordinary skill in the art, the specific meaning of the above terms in the present utility model shall be understood as the case may be.

Unless defined otherwise, all technical and scientific terms used herein shall be understood as the same meaning with those commonly accepted by those of ordinary skill in the art. All technical and scientific terms used in the description of the present utility model are merely for the purpose of illustrating specific embodiments only, and are not intended to limit the present utility model. As used herein, the term "and/or" encompasses any and all combinations of one or more related listed items.

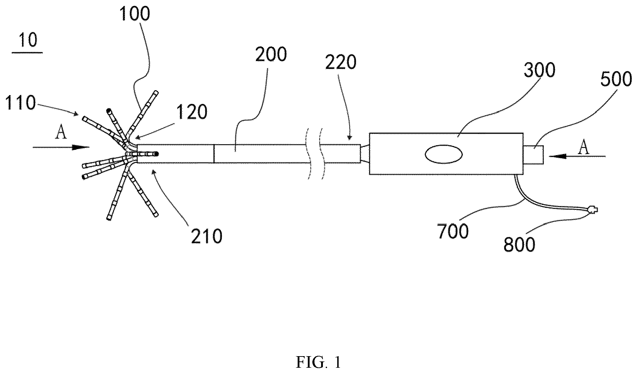

Referring to FIG. 1, an electrophysiological mapping catheter device is provided in an embodiment of the present utility model.

An electrophysiological mapping catheter device 10 includes a catheter, an adjusting handle device 300, electrodes 400, and a connector 500. The catheter includes branch tubes 100 and an adjusting tube 200.

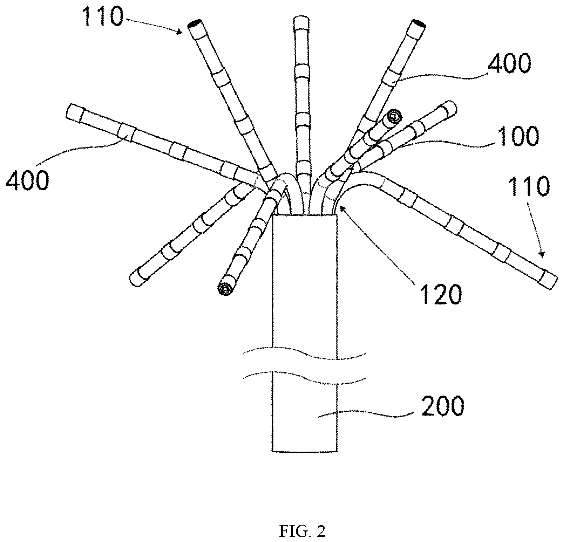

Referring to FIG. 1, there are multiple branch tubes 100. The two ends of each branch tube 100 are respectively a detection end 110 and a connection end 120. The plurality of the detection ends 110 are arranged in a staggered manner. Referring to FIG. 2, an electrode 400 is disposed on each detection end 110. The connection ends 120 are connected to the adjusting tube 200.

The adjusting tube 200 is flexible. The adjusting tube 200 is also connected to the adjusting handle device 300. The bending angle of the adjusting tube 200 can be adjusted by the adjusting handle device 300.

The connector 500 is disposed on the adjusting handle device 300 and is electrically connected to the electrodes 400. The connector 500 is further configured to be connected with an electrophysiological system for transmitting electrical signals detected by the detection ends 110.

In a specific embodiment, the end surfaces of the plurality of the detection ends 110 are disposed at different locations of the same curved surface. It should be noted that the curved surface is a virtual surface which is composed of the end surfaces of the plurality of the detection ends 110. When the curved surface is a spherical surface, the spherical surface is a circumscribed spherical surface of the end surfaces of the plurality of the detection ends 110.

In a specific embodiment, the curved surface can be a spherical surface, a partial spherical surface, an elliptical surface, or a partial elliptical surface. For example, in an embodiment, all detection ends 110 are distributed at different locations of the same spherical surface. In another embodiment, all detection ends 110 are distributed at different locations of the same hemispherical surface.

In a specific embodiment, the plurality of the branch tubes 100 are divided into at least two groups, and the branch tubes 100 in each group are symmetrically distributed with respect to the axial direction of the adjusting tube 200. Specifically, the branch tubes 100 in each group are circumferentially and symmetrically distributed with respect to the axial direction of the adjusting tube 200, that is, the branch tubes 100 in each group are distributed in the same circumferential surface with the axis of the circumferential surface as its symmetric axis. For example, the branch tubes 100 in each group can be symmetrically distributed in an umbrella-shape. The branch tubes 100 can be 2 to 16 in number and can be divided into 2 to 8 groups, and the branch tubes 100 in each group can be circumferentially and symmetrically distributed with respect to the axial direction of the adjusting tube 200. For example, in an embodiment, the number of the branch tubes 100 is 9, the nine branch tubes 100 are divided into three groups, and the three branch tubes 100 in each group are circumferentially distributed. It can be understood that in other embodiments, the number of the branch tubes 100 can be varied, and the number of the branch tubes 100 in each group can be 2, 4, etc., which is not enumerated herein.

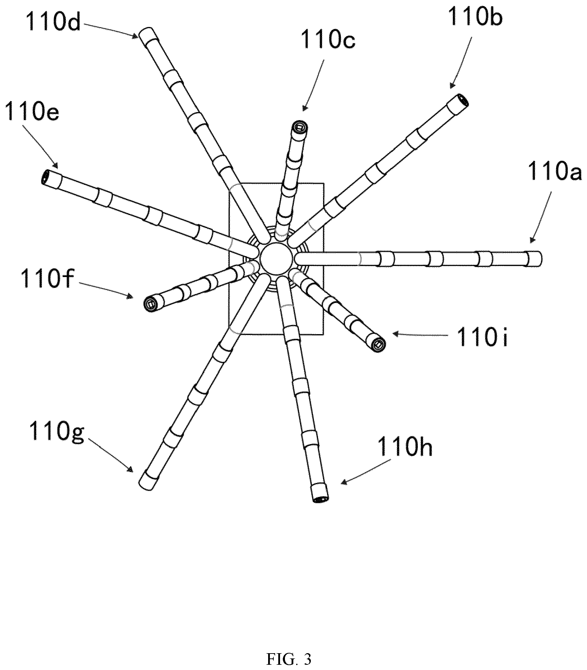

In a specific embodiment, referring to FIGS. 2 and 3, nine branch tubes 100 are shown in this embodiment. The nine branch tubes 100 are divided into three groups, and thus there are three branch tubes 100 in each group. In the counterclockwise direction, the detection ends 110 of the nine branch tubes 100 are sequentially numbered as 110a, 110b, 110c, 110d, 110e, 110f, 110g, 110h, and 110i. The detection ends 110a, 110b, 110c, 110d, 110e, 11 0f, 110g, 110h, and 110i of the nine branch tubes 100 are symmetrically distributed with respect to the axial direction. The detection ends 110a, 110d, and 110g are divided into one group and have an axial angle with the adjusting tube 200 of 60°; the detection ends 110b, 110e, and 110h are divided into one group and have an axial angle with the adjusting tube 200 of 110°; and the detection ends 110c, 110f, and 110i are divided into one group and have an axial angle with the adjusting tube 200 of 150°. In this embodiment, the lengths of all branch tubes 100 are the same, and the detection ends of the nine branch tubes 100 integrally circumscribe a spherical structure, forming a dandelion-like shape.

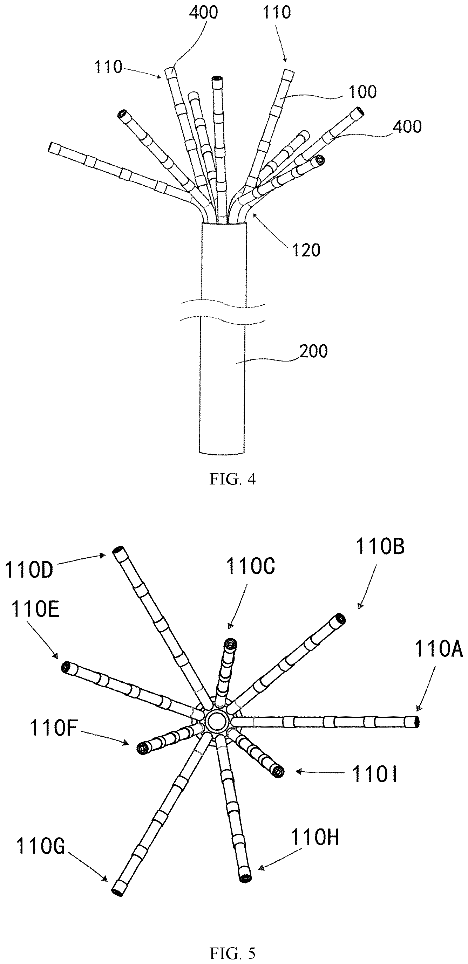

In another specific embodiment, referring to FIGS. 4 and 5, nine branch tubes 100 are shown in this embodiment. The nine branch tubes 100 are divided into three groups, and thus there are three branch tubes 100 in each group. In the counterclockwise direction, the detection ends 110 of the nine branch tubes 100 are sequentially numbered as 110A, 110B, 110C, 110D, 110E, 110F, 110G, 110H, and 110I. The detection ends 110A, 110B, 110C, 110D, 110E, 110F, 110G, 110H, and 110I of the nine branch tubes 100 are symmetrically distributed with respect to the axial direction. The detection ends 110A, 110D, and 110G are divided into one group and have an axial angle with the adjusting tube 200 of 110°; the detection ends 110B, 110E, and 110F are divided into one group and have an axial angle with the adjusting tube 200 of 130°; and the detection ends 110C, 110F, and 110I are divided into one group and have an axial angle with the adjusting tube 200 of 160°. In this embodiment, the lengths of all branch tubes 100 are the same, and the detection ends of the nine branch tubes 100 integrally circumscribe a hemispherical structure.

In a specific embodiment, the axial angle between the branch tubes 100 and the adjusting tube 200 is 10° to 180°. For example, in an embodiment, the axial angle between the branch tube 100 and the adjusting tube 200 is 10°. In another embodiment, the axial angle between the branch tube 100 and the adjusting tube 200 is 90°. In yet another embodiment, the axial angle between the branch tube 100 and the adjusting tube 200 is 160°. The axial angle between the branch tube 100 and the adjusting tube 200 can be adjusted as actual needs.

In a specific embodiment, the branch tubes 100 are flexible. The branch tubes 100 can be gathered by applying an external force and can be restored in shape when the external force is removed. The detection ends 110 of the branch tubes 100 can be overall flexible and have a relatively high elasticity. Furthermore, the branch tubes 100 can have shape memory capability. The detection ends 110 can be retracted into a sheath. When pushed out from the sheath, the detection ends 110 can be naturally diverged, forming a preset shape such as a spherical shape, a hemispherical shape, or an elliptical shape.

In a specific embodiment, the adjusting tube 200 includes a distal end portion 210 connected to the branch tubes 100 and a proximal end portion 220 connected to the adjusting handle device 300. The electrophysiological mapping catheter device 10 further includes an adjusting pulling wire. One end of the adjusting pulling wire is connected to the distal end portion 210, and the other end of the adjusting pulling wire is connected to the adjusting handle device 300. The adjusting pulling wire can be driven to move by manipulating a controller disposed in the adjusting handle device 300. The movement of the adjusting pulling wire can drive the bending of the distal end portion 210, so as to adjust the bending angle of the distal end portion 210.

In a specific embodiment, the distal end portion 210 is flexible. The distal end portion 210 can be bent by adjusting the adjusting handle device 300 as required.

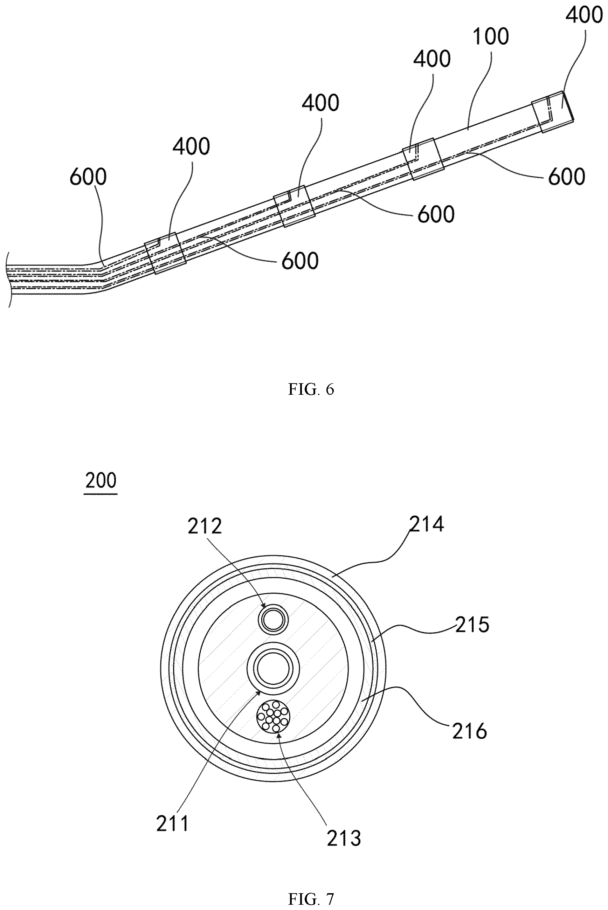

Referring to FIG. 7, in a specific embodiment, the distal end portion 210 includes a first tube chamber 211. One opening end of the first tube chamber 211 is disposed at the distal end portion 210 and the other end of the first tube chamber 211 is in communication with a perfusion tube disposed on the side or end of the adjusting handle device 300. The end of the perfusion tube is connected to a Luer taper 800. When use, the perfusion tube is connected to the Luer taper 800, and can perfuse liquid at a certain flow rate. The perfused liquid can be drained out from the opening of the first tube chamber 211 located at the distal end portion 210.

In a specific embodiment, the perfusion tube can be extended to the adjusting handle device 300 and in communication with an extension tube 700. The extension tube 700 is connected to the adjusting handle device 300 and is configured for connecting with an external perfusion pump.

Referring to FIG. 7, in a specific embodiment, the distal end portion 210 includes a second tube chamber 212. One end of the second tube chamber 212 is in communication with the distal end portion 210 and the other end of the second tube chamber 212 is in communication with the proximal end portion 220. One end of the adjusting pulling wire is connected to the distal end portion 210, and the other end of the adjusting pulling wire is passed through the second tube chamber 212 and connected to the adjusting handle device 300.

Referring to FIG. 7, in a specific embodiment, the distal end portion 210 further includes a third tube chamber 213. One end of the third tube chamber 213 is in communication with the branch tubes 100 and the other end of the third tube chamber 213 is in communication with the proximal end portion 220. The conducting wires 600 of the electrodes 400 are sequentially passed through the third tube chamber 213, the proximal end portion 220, and the adjusting handle device 300 and connected to the connector 500.

Further, the adjusting tube 200 can be a three-layer structure, including an outer tube 214, an intermediate reinforcement metal layer 215, and an inner tube 216. The first tube chamber 211, the second tube chamber 212 and the third tube chamber 213 are disposed in the inner tube 216.

In a specific embodiment, the branch tubes 100 can be made of one or more materials selected from the group consisting of polyimide, polyether, polyester, nylon, and copolymers thereof. The branch tubes 100 can be a thin-wall tube with a single chamber, without an embedded reinforcement metal layer.

A stainless-steel wire mesh is embedded in the adjusting tube 200, so that the branch tubes 100 have good twist control, bending resistance and compliance. Stainless-steel wire mesh is knotted using 16 or 32 stainless-steel wires with a diameter of 0.02 to 0.1 mm. The pore density of the stainless-steel wire mesh is 20 to 100 PPI.

In a specific embodiment, the adjusting tube 200 is made of one or more materials selected from the group consisting of polyimide, polyether, polyester, nylon, and copolymers thereof.

Referring to FIG. 6, in a specific embodiment, the electrodes 400 can be ring-shaped electrodes 400. The width of the ring-shaped electrode 400 is 0.2 to 2 mm. The outer diameter of the ring-shaped electrode 400 is 0.3 to 1 mm. The distance between the adjacent ring-shaped electrodes 400 is 1 to 10 mm. The inner surfaces of the ring-shaped electrodes 400 are respectively connected to the conducting wires 600. The conducting wires 600 are insulated from each other and connected to the connector 500.

The surface of the detection end 110 for fixing the electrode 400 is provided with a hole, allowing one end of the conducting wire 600 to pass therethrough and be electrically connected with the inner wall of the electrode 400.

Further, the conducting wire 600 can be an enameled wire with a core made of any high-conductivity metal such as copper, gold, silver, nickel, etc., and an insulating layer coated at an outer surface. The diameter of the conducting wire 600 is 0.05 to 0.20 mm. The insulating layer is made of one or more materials selected from the group consisting of polyimide resins, polyurethane resins, polyester resins, polyester-imides, polyamide-imides, and composite polyester-imides.

Preferably, in an embodiment, the electrode 400 is processed from a material such as platinum, platinum-iridium alloy, gold, etc.

In a specific embodiment, the ring-shaped electrodes 400 are equally spaced.

Use of the electrophysiological mapping catheter device 10 of the present utility model includes the following steps:
(1) The femoral vein on one side is punctured, and a guidewire catheter is delivered through the femoral vein.
(2) A transseptal puncture sheath is introduced to puncture the atrial septum.
(3) The electrophysiological mapping catheter device 10 is introduced into the heart chamber through the puncture sheath. The connector 500 is connected to an electrophysiological system such as a multi-lead recorder. Then the electrophysiological mapping catheter device 10 is continued to be pushed forward, such that the electrodes 400 on the branch tubes 100 are in contact with the endocardial surface, and the detection ends 110 of the branch tubes 100 and the electrodes 400 on the outer wall thereof can be extended into folds or sulci in the pectinate muscles or trabeculae muscles in the heart chamber. Meanwhile, the bending shape of the distal end portion 210 of the adjusting tube 200 can be varied by adjusting the adjusting handle device 300, so that the electrodes 400 on the detection ends 110 of the branch tubes 100 can be in contact with the wall of the heart chamber more sufficiently.
(4) The electrophysiological signals from the heart chamber can be received and transmitted by the electrodes 400 on the detection ends 110 of the branch tubes 100. The location of the lesion can be identified by the surgeons via observing the electrocardiogram, thereby assisting the subsequent ablation procedure.
(5) An ablation operation is performed.
(6) After the ablation, the intracardiac mapping of step (3) is performed again. The catheter can be withdrawn if the lesion is completely eliminated. If the lesion is not ablated completely, the ablation procedure is performed again until the lesion is totally removed.

The electrophysiological mapping catheter device 10 of the present utility model can adapt to the irregular heart chamber space and record electrophysiological signals from numerous folds or loose sulci on the endocardial surface, and can form a more accurate three-dimensional mapping image in a relatively short time, reducing the difficulty of identification by surgeons and facilitating the operation. The electrophysiological mapping catheter device 10 of the present utility model can make sufficient contact with an irregular heart chamber structure, a non-smooth inner wall, as well as a narrow space at the proximal or distal end, etc., and thus can accurately record the electrical signals. The plurality of branch tubes 100 of the electrophysiological mapping catheter device 10 of the present utility model form a divergent spatial structure with the detection ends 110 as arranged in a staggered manner. The staggered detection ends 110 can adapt to various irregular heart chamber structures, and the electrodes 400 of the detection ends 110 can readily access to various narrow spaces and microstructures in the heart chamber.

Compared with the prior art, the plurality of branch tubes 100 of the electrophysiological mapping catheter device 10 form a divergent structure with adjustable angles. The numbers of the branch tubes 100 and the electrodes 400 are relatively large. Therefore, the reliability of the contact between the electrodes 400 and the irregular heart chamber, or between the electrodes 400 and folds or sulci on the inner wall of the heart chamber during the operation is improved, and thus the precision and accuracy of electrophysiological signal acquisition are improved, thereby reducing the operation time, making the operation safer and more efficient, and providing great convenience for the surgeons.

The technical features of the above-mentioned embodiments can be combined arbitrarily. In order to make the description concise, not all possible combinations of the technical features are described in the embodiments. However, as long as there is no contradiction in the combination of these technical features, the combinations should be considered as in the scope of the present disclosure.

The above-described embodiments are only several implementations of the present utility model, and the descriptions are relatively specific and detailed, but they should not be construed as limiting the scope of the present utility model. It should be understood by those of ordinary skill in the art that various modifications and improvements can be made without departing from the concept of the present utility model, and all fall within the protection scope of the present utility model. Therefore, the patent protection of the present utility model shall be defined by the appended claims.

## Claims

1. An electrophysiological mapping catheter device, comprising a catheter, an adjusting handle device, electrodes, and a connector, wherein the catheter comprises an adjusting tube and a plurality of branch tubes, two ends of each branch tube are respectively a connection end and a detection end, the plurality of detection ends are arranged in a staggered manner, the electrodes are respectively disposed on the plurality of detection ends, the plurality of connection ends are connected to the adjusting tube, the adjusting tube is connected to the adjusting handle device, the adjusting handle device is capable of adjusting a bending angle of the adjusting tube, the connector is disposed on the adjusting handle device and is electrically connected to the electrodes, and the connector is further configured to be connected with an electrophysiological system for transmitting electrical signals detected by the electrodes.

2. The electrophysiological mapping catheter device according to claim 1, wherein end surfaces of the plurality of detection ends of the plurality of branch tubes are disposed in a same curved surface.

3. The electrophysiological mapping catheter device according to claim 2, wherein the curved surface is a spherical surface, a partial spherical surface, an elliptical surface, or a partial elliptical surface.

4. The electrophysiological mapping catheter device according to any one of claims 1 to 3, wherein an axial angle between the plurality of branch tubes and the adjusting tube is 10° to 180°.

5. The electrophysiological mapping catheter device according to any one of claims 1 to 3, wherein the plurality of branch tubes are flexible and have shape memory capability.

6. The electrophysiological mapping catheter device according to any one of claims 1 to 3, wherein the plurality of branch tubes are divided into at least two groups, and the branch tubes in each group are symmetrically distributed with respect to an axial direction of the adjusting tube.

7. The electrophysiological mapping catheter device according to claim 6, further comprising an adjusting pulling wire, wherein the adjusting tube comprises a distal end portion connected to the plurality of branch tubes and a proximal end portion connected to the adjusting handle device, one end of the adjusting pulling wire is connected to the distal end portion, and another end of the adjusting pulling wire is connected to the adjusting handle device.

8. The electrophysiological mapping catheter device according to claim 7, wherein the distal end portion is flexible.

9. The electrophysiological mapping catheter device according to claim 7, wherein the distal end portion comprises a first tube chamber, one opening end of the first tube chamber is disposed at an end surface of the distal end portion, and another end of the first tube chamber is in communication with a perfusion tube disposed in the proximal end portion, the perfusion tube is extended to the adjusting handle device and is in communication with an extension tube, and the extension tube is connected to the adjusting handle device and is configured for attaching with an external perfusion pump.

10. The electrophysiological mapping catheter device according to claim 7, wherein the distal end portion comprises a second tube chamber, one end of the second tube chamber is in communication with the distal end portion, and another end of the second tube chamber is in communication with the proximal end portion; one end of the adjusting pulling wire is connected to the distal end portion, and another end of the adjusting pulling wire is passed through the second tube chamber and connected to the adjusting handle device.

11. The electrophysiological mapping catheter device according to claim 7, wherein the distal end portion further comprises a third tube chamber, one end of the third tube chamber is in communication with the branch tubes, and another end of the third tube chamber is in communication with the proximal end portion, conducting wires of the electrodes are sequentially passed through the third tube chamber, the proximal end portion, and the adjusting handle device, and connected to the connector.

12. The electrophysiological mapping catheter device according to any one of claims 1 to 3, and 7 to 11, wherein the electrodes are ring-shaped electrodes, the ring-shaped electrodes each have a width of 0.2 to 2 mm and an outer diameter of 0.3 to 1 mm, a distance between adjacent ring-shaped electrodes is 1 to 10 mm, inner surfaces of the ring-shaped electrodes are respectively connected to conducting wires, and the conducting wires are insulated from each other and connected to the connector.
